# EUROPEAN PATENT APPLICATION

(11) **EP 4 782 472 A1**
(43) Date of publication of application: **29.07.2026**
(21) Application number: 24868014.2
(22) Date of filing: 22.08.2024
(51) Int. Cl.: C08F 292/00, A61K 6/62, A61K 6/70, A61K 6/887, B29C 64/124, B29C 64/314, B33Y 70/10, C08F 2/44, C08F 2/50, C08F 20/00, C08F 257/00, C08F 265/06, C08F 290/06, C08K 3/013, C08L 51/10

(54) **THREE-DIMENSIONAL STEREOLITHOGRAPHIC CURABLE COMPOSITION, METHOD FOR PRODUCING SAME, AND METHOD FOR PRODUCING THREE-DIMENSIONAL STEREOLITHOGRAPHIC ARTICLE**

(30) Priority: 22.09.2023 JP 2023158707
(71) Applicant: Tokuyama Dental Corporation, Tokyo 110-0016 (JP)
(72) Inventor: GYAKUSHI Ayumu, Tokyo 110-0016 (JP); SAKATA Eibu, Tokyo 110-0016 (JP); NAKASHIMA Kei, Tokyo 110-0016 (JP)
(74) Representative: HGF
(86) International application number: PCT/JP2024/029794
(87) International publication number: WO 2025/062934

(57) **Abstract**

Provided are a curable composition for three-dimensional photofabrication and a method for producing the same, the curable composition for three-dimensional photofabrication comprising: 100 parts by mass of a radically-polymerizable monomer (A) that is liquid at 25°C; 50 to 250 parts by mass of an inorganic filler (B); 0.05 to 5.0 parts by mass of a photopolymerization initiator (C) that absorbs activating light and generates radicals; 0.01 to 2.5 parts by mass of an activating light absorbent (D) that absorbs the activating light; 0.01 to 5.0 parts by mass of a polymerization inhibitor (E); and 0.3-10 parts by mass of a hyperbranched polymer (F) that is soluble in the radically-polymerizable monomer (A). Moreover, provided is a method for producing a three-dimensional photofabricated product using said for three-dimensional photofabrication curable composition for three-dimensional photofabrication.

## Description

### TECHNICAL FIELD

The present disclosure relates to a curable composition for three-dimensional photofabrication, a method for producing the same, and a method for producing a three-dimensional photofabricated product.

### BACKGROUND ART

A technique for shaping a three-dimensional fabricated product by irradiating a photocurable composition (also referred to as photocurable resin or photocurable resin composition) containing a polymerizable monomer and a photopolymerization initiator with light (activating light) for activating the photopolymerization initiator to cure the photocurable composition is known as a photofabrication method. Photofabrication includes several methods. Above all, vat photopolymerization methods are widely used because the devices are relatively inexpensive and fabricated products with smooth surfaces can be produced with high accuracy.

In a vat photopolymerization method, a three-dimensional object as a desired product is generally obtained as follows. First, from three-dimensional shape data indicating a shape of a three-dimensional object, height directions of the three-dimensional object are digitized and serialized, and two-dimensional shape data indicating sectional shapes of the three-dimensional object at the serialized heights is generated. Subsequently, a liquid photocurable composition contained in the vat is irradiated with activating light at positions predetermined based on the two-dimensional shape data so that the liquid photocurable composition located at the positions is selectively primarily-cured to form shaped layers having the sectional shapes, and shaped layers having sectional shapes at the heights are sequentially formed in accordance with the order of the serialization and stacked to obtain a stacked body having a shape corresponding to the shape of the three-dimensional object. Subsequently, if necessary, the stacked body is washed with an organic solvent and then secondarily-cured to obtain a desired article.

In the dental field, dental restorations such as dentures and crown prostheses having shapes specific to each patient's oral condition need to be produced with high dimensional accuracy. Thus, it has been investigated to produce dental restorations by photofabrication using a vat photopolymerization method based on a computer aided design (CAD) data designed from digital data acquired via intraoral scanning or the like.

Dental prostheses used in the oral cavity require not only the high dimensional (shape) accuracy described above but also high mechanical strength sufficient to withstand loads during mastication. In this regard, when blending an inorganic filler into the photocurable composition, polymerization shrinkage as a cause of reduced accuracy can be suppressed, and the mechanical strength and surface hardness of the cured body can be improved. For this reason, it is considered that a vat photopolymerization method using a photocurable composition containing an inorganic filler is suitable for photofabrication of dental prostheses. Such a curable composition for three-dimensional photofabrication containing an inorganic filler has also been proposed.

For example, Patent Document 1 discloses a composition containing a polymerizable monomer (a), an UV absorbable inorganic particle (b), and a photopolymerization initiator (c), as a composition for optical fabrication excellent in shaping accuracy, dynamic characteristics, and transparency and suitable particularly as dental materials. Furthermore, Patent Document 2 discloses a composition containing a translucent resin and two or more translucent particles having different refractive indexes and Abbe numbers, as a resin composition that enables preparation of a cured body (three-dimensional fabricated product) having excellent designability. Furthermore, Patent Document 3 discloses a composition containing a urethanized (meth)acrylic compound (a), a (meth)acrylamide compound (b), a photopolymerization initiator (c), and a spherical inorganic particle (d) in a content of 50 to 400 parts by mass with respect to the total of 100 parts by mass of the polymerizable monomer and having an average particle diameter of 0.75 to 10 µm, as a resin composition for photofabrication that enables formation of a three-dimensional fabricated product having high strength, high elasticity, and excellent wear resistance.

Patent Document 4 discloses a "composition that characteristically contains a photocurable viscous mixture containing 0 to 50 wt% of a solution of polymerized poly(methyl methacrylate) dissolved in a methyl methacrylate monomer solvent, 5 to 20 wt% of at least one polyfunctional aliphatic (meth)acrylate, 5 to 40 wt% of at least one aliphatic urethane (meth)acrylate oligomer, 25 to 65 wt% of at least one bifunctional bisphenol-A dimethacrylate, 0.1 to 5 wt% of at least one photoinitiator, 0.05 to 2 wt% of at least one light stabilizer, and 0.1 to 3 wt% of a coloring pigment based on a total weight of the composition", as a composition suitable for producing artificial teeth and denture bases using a three-dimensional printing method and having low viscosity and low shrinkage property.

### Citation List

### Patent Documents

Patent Document 1: PCT International Publication No. WO 2018/074380
Patent Document 2: Japanese Unexamined Patent Application, Publication No. 2020-180171
Patent Document 3: Japanese Unexamined Patent Application, Publication No. 2022-41276
Patent Document 4: Japanese Unexamined Patent Application, Publication No. 2016-525150
Patent Document 5: Japanese Patent No. 5882921

### DISCLOSURE OF THE INVENTION

### Problems to be Solved by the Invention

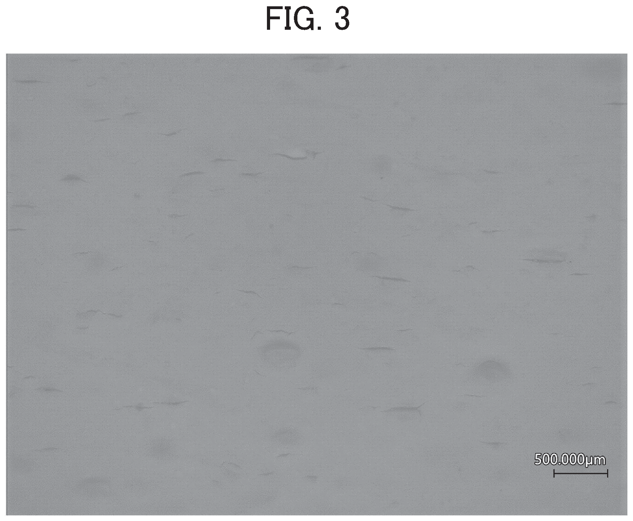

In Patent Documents 1 to 3, a composition with a polymerizable monomer containing an inorganic filler is used, and therefore a fabricated product having a preferable mechanical strength, elastic modulus, and wear resistance can be obtained. However, since the composition for optical fabrication described in Patent Document 1 has low fluidity, shapes of an applicable fabricated product may be restricted in some cases. Furthermore, when the resin composition described in Patent Document 2 is left to stand for a long period of time, translucent particles (e.g. glass filler) contained in the composition may settle in some cases. Furthermore, it has been proved that the resin composition for photofabrication described in Patent Document 3 frequently develops visually-unrecognizable fine cracks on the surface of the fabricated product (see FIG. 3). Use of such fabricated products with fine cracks as dental prostheses is problematic because the cracks may cause fracture of the dental prostheses in an oral cavity.

When a polymerized poly(methyl methacrylate) is added to the composition described in Patent Document 4, shrinkage of the fabricated product during the photofabrication is reduced, and cracking on the surface of the fabricated product is suppressed. However, the mixing of such a polymer component into the composition tends to reduce the strength of the fabricated product. Furthermore, since the viscosity of the composition tends to increase, handleability of the composition is deteriorated because the viscosity is considerably increased when an inorganic filler is blended for enhancing the strength.

Thus, an object of the present disclosure is to provide a technology that makes it possible to produce a high-strength three-dimensional photofabricated product without developing cracks on a surface of the product when producing the three-dimensional photofabricated product by photofabrication of a curable composition for three-dimensional photofabrication containing a certain amount of an inorganic filler for strength enhancement using a vat photopolymerization method.

### Means for Solving the Problems

Specific means for solving the above problems includes the following aspects.
<1> A curable composition for three-dimensional photofabrication containing:
   100 parts by mass of a radically-polymerizable monomer (A) that is liquid at 25°C;
   50 to 250 parts by mass of an inorganic filler (B);
   0.05 to 5.0 parts by mass of a photopolymerization initiator (C) that absorbs activating light to generate radicals;
   0.01 to 2.5 parts by mass of an activating light absorbent (D) that absorbs the activating light;
   0.01 to 5.0 parts by mass of a polymerization inhibitor (E); and
   0.3 to 10 parts by mass of a hyperbranched polymer (F) that is soluble in the radically-polymerizable monomer (A).
<2> The curable composition for three-dimensional photofabrication according to <1>, in which the hyperbranched polymer (F) includes:
   a first unit structure having four bonding sites and
   represented by the following formula (I): 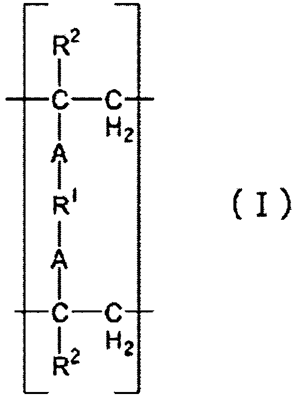
   wherein A each independently represent a single bond for bonding C and R¹, >C=O, -O-, -COO-, or -COO-CH₂-, R¹ represents a divalent saturated aliphatic hydrocarbon group or a divalent aromatic hydrocarbon group, and R² each independently represent hydrogen atom or a methyl group; and
   a second unit structure that is at least one of two unit structures represented by the following formulas (IIA) and (IIB): 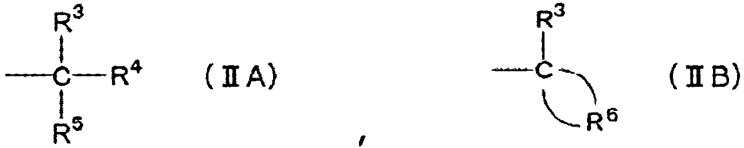
   wherein R³, R⁴, and R⁵ each independently represent hydrogen atom, an alkyl group having 1 to 5 carbon atoms for forming a main chain, an alkoxycarbonyl group having 2 to 6 carbon atoms for forming a main chain, an aryl group, or a cyano group, and R⁶ represents an alkylene group having 4 to 10 carbon atoms for forming a main chain.
<3> The curable composition for three-dimensional photofabrication according to <1> or <2>, in which a content of the hyperbranched polymer (F) is 2 to 10 parts by mass with respect to 100 parts by mass of the radically-polymerizable monomer (A).
<4> The curable composition for three-dimensional photofabrication according to any one of <1> to <3>, in which 90 mass% or more of the radically-polymerizable monomer (A) is composed of a polyfunctional (meth)acrylate type radically-polymerizable monomer (a) having two or more (meth)acryloyl groups in its molecule and having 5 to 25 atoms constituting a main chain of a divalent group interposed between the two or more (meth)acryloyl groups present in its molecule, the polyfunctional (meth)acrylate type radically-polymerizable monomer (a) is composed of a polyfunctional (meth)acrylate type radically-polymerizable monomer (a1) having at least one selected from a group consisting of a urethane bond, a urea bond, and an isocyanurate ring; and another polyfunctional (meth)acrylate type radically-polymerizable monomer (a2), and 40 to 90 mass% of the radically-polymerizable monomer (A) is the polyfunctional (meth)acrylate type radically-polymerizable monomer (a1), and 10 to 60 mass% of the radically-polymerizable monomer (A) is the polyfunctional (meth)acrylate type radically-polymerizable monomer (a2).
<5> The curable composition for three-dimensional photofabrication according to any one of <1> to <4>, additionally containing 0.005 to 1.0 part by mass of chain transfer agent (G).
<6> The curable composition for three-dimensional photofabrication according to any one of <1> to <5>, which is for use as a liquid photocurable resin composition in a three-dimensional photofabrication in which, from three-dimensional shape data indicating a shape of a three-dimensional object, a height direction of the three-dimensional object is digitized and serialized, and two-dimensional shape data indicating sectional shapes of the three-dimensional object at serialized heights is generated, and then, using a vat photopolymerization method including irradiating a predetermined position of the liquid photocurable resin composition in a vat with activating light that is ultraviolet ray or visual light to selectively cure the liquid photocurable resin composition at the position, fabricated layers having shapes corresponding to the two-dimensional shapes at the heights are sequentially formed and stacked in accordance with the order of the serialization on the basis of the two-dimensional shape data, thereby obtaining a stacked body having a shape corresponding to the shape of the three-dimensional object.
<7> A method for producing the curable composition for three-dimensional photofabrication according to any one of <1> to <6>, the method including:
   mixing the radically-polymerizable monomer (A), the photopolymerization initiator (C), the activating light absorbent (D), the polymerization inhibitor (E), and the hyperbranched polymer (F) to prepare a liquid composition containing at least a part of the hyperbranched polymer dissolved therein, and
   mixing the liquid composition and the inorganic filler (B).
<8> The method for producing the curable composition for three-dimensional photofabrication according to <7>, in which, when preparing the liquid composition, a hyperbranched polymer powder granule having a hydrodynamic average diameter of 2 to 40 nm as measured in tetrahydrofuran by a dynamic light scattering method is mixed.
<9> A method for producing a three-dimensional photofabricated product by using the curable composition for three-dimensional photofabrication according to any one of <1> to <6> as a liquid photocurable resin composition, the method including
   a forming step in which, from three-dimensional shape data indicating a shape of a three-dimensional object, a height direction of the three-dimensional object is digitized and serialized, and two-dimensional shape data indicating sectional shapes of the three-dimensional object at serialized heights is generated, and then, using a vat photopolymerization method including irradiating a predetermined position of the liquid photocurable resin composition in a vat with activating light that is ultraviolet ray or visual light to selectively cure the liquid photocurable resin composition at the position, fabricated layers having shapes corresponding to the two-dimensional shapes at the heights are sequentially formed and stacked in accordance with the order of the serialization on the basis of the two-dimensional shape data, thereby obtaining a stacked body having a shape corresponding to the shape of the three-dimensional object.

### Effects of the Invention

The curable composition for three-dimensional photofabrication according to the present disclosure makes it possible to produce a three-dimensional photofabricated product having excellent mechanical strength and good shaping accuracy while suppressing decrease in fluidity and sedimentation of inorganic fillers and preventing cracking of a cured product surface.

### BRIEF DESCRIPTION OF THE DRAWINGS

FIG. 1 is a diagram presenting a surface of a three-dimensional photofabricated product obtained in Example 1 as observed under an optical microscope (magnification: 50X);
FIG. 2 is a diagram presenting a surface of a three-dimensional photofabricated product obtained in Comparative Example 1 as observed under an optical microscope (magnification: 50X); and
FIG. 3 is a diagram presenting a surface of a three-dimensional photofabricated product obtained in Comparative Example 5 as observed under an optical microscope (magnification: 50X).

### PREFERRED MODE FOR CARRYING OUT THE INVENTION

As described above, the photocurable composition containing a certain amount of inorganic filler for improving the mechanical strength and surface hardness of the cured body has problems of reduced fluidity and particle sedimentation during its storage in some cases. Furthermore, when a fabricated product is produced using a vat photopolymerization method with the resin composition for photofabrication described in Patent Document 3, visually-unrecognizable fine cracks are generated on the surface of the shaped article in many cases (although the cracks are not recognized in Patent Document 3).

Although the reason for the cracking on the surface of the fabricated product is not clear, the present inventors have confirmed that cracks are generated when washing uncured curable compositions adhering to the surface of the stacked body with an organic solvent after obtaining the stacked body using a photofabrication apparatus. The bonded portions between fabricated layers in the stacked body exhibit a lower polymerization rate than of other portions. When the stacked body contains an inorganic filler, portions where a laser light irradiation intensity is weakened due to light scattering have a lower polymerization rate than of other portions. When an organic solvent for washing permeate the regions with a lower polymerization rate, the regions swell, thereby the intermolecular distances between the polymer chains constituting the formed body widen, and the strength of the formed body is temporarily decreased. In this process, it is considered that, in the forming step, an internal stress remaining in the formed body destroys portions with decreased strength to cause cracks in the regions.

Although the reason why cracks on the surface of the shaped article are not recognized in Patent Document 3 is unclear, the present inventors have observed cracks in a supplementary experiment. This experiment suggests that the cracking was overlooked in Patent Document 3.

For the purpose of enhancing the strength of the fabricated product while avoiding problems of reduced fluidity and particle sedimentation during storage and suppressing cracking, the content of the inorganic filler is set to 50 to 250 parts by mass with respect to 100 parts by mass of the polymerizable monomer, and a hyperbranched polymer soluble in the polymerizable monomer is blended in the curable composition for three-dimensional photofabrication according to the present disclosure.

Although the reason why the cracking can be suppressed by blending a certain amount of hyperbranched polymer is unclear, the reason is assumed as follows. In other words, hyperbranched polymers have a structural feature that their repeating units contain branching points, and their molecular chains spread three-dimensionally (in a dendritic manner). For this reason, it is considered that the surface of the stacked body can be protected while holding the polymerizable monomer thereinside and swelling of the stacked body due to the organic solvent during the washing can be prevented, by blending the hyperbranched polymer. Furthermore, it is considered that internal stresses generated in the stacked body during curing can be alleviated by blending the hyperbranched polymer.

It is known that a hyperbranched polymer is blended into a dental curable composition such as a dental composite resin to suppress polymerization shrinkage during dental cavity restoration (see Patent Document 5). However, such a dental curable composition has a relatively high polymerization shrinkage rate of about 2% even with a significantly high inorganic filler content of 270 parts by mass with respect to 100 parts by mass of the polymerizable monomer. In the three-dimensional stereolithography using a vat photopolymerization method, polymerization occurs in a state that unpolymerized materials are present in the surrounding area, and therefore the polymerization shrinkage is not considered to immediately lead to cracking. Thus, the above technologies do not suggest the effects of the present invention.

Regarding the agglomeration and sedimentation of the inorganic fillers during storage, it is considered that the inorganic fillers can be prevented from aggregating and settling from each other by fine hyperbranched polymers sufficiently dissolved and uniformly dispersed in the curable composition for three-dimensional photofabrication according to the present disclosure.

Since the hyperbranched polymer exhibits small intermolecular entanglement, behaves like fine particles, and finely disperses in the composition, the viscosity of the composition is hard to increase. Furthermore, since the polymerizable monomer contained in the hyperbranched polymer ultimately cures, the strength of the resulting three-dimensional photofabricated product is hard to decrease. When blending a polymer other than the hyperbranched polymer, such as a poly(meth)acrylate, the increased viscosity of the composition tends to lead to not only decrease in the fluidity but also decrease in the strength of the resulting three-dimensional photofabricated product.

The curable composition for three-dimensional photofabrication (hereinafter also referred to simply as "curable composition for photofabrication") according to the present disclosure, the method for producing the composition, and the method for producing the three-dimensional photofabricated product will be explained below. Unless otherwise specified herein, the notation "x to y" using numerical values x and y signifies "x or more and y or less". When a unit is attached only to the numerical value y in this notation, the unit is supposed to be also applicable to the numerical value x. Furthermore, in this specification, the term "(meth)acryloyl" means both "acryloyl" and "methacryloyl". Similarly, the term "(meth)acrylate" means both "acrylate" and "methacrylate".

### <Curable Composition for Photofabrication>

The curable composition for photofabrication according to the present disclosure refers to a curable composition usable as a liquid photocurable composition for producing a three-dimensional photofabricated product using a vat photopolymerization method with activating light including a light at a specific wavelength λ (nm) in the ultraviolet or visible light region, i.e. a curable composition for three-dimensional photofabrication by a vat photopolymerization method.

Herein, the vat photopolymerization method means a method for forming a three-dimensional photofabricated product having a shape corresponding to a shape of a three-dimensional object, the method including a step in which, from three-dimensional shape data indicating a shape of a three-dimensional object, a height direction of the three-dimensional object is digitized and serialized, and two-dimensional shape data indicating sectional shapes of the three-dimensional object at the serialized heights is generated, subsequently, a liquid photocurable composition contained in the vat is irradiated with activating light at positions predetermined based on the two-dimensional shape data so that the liquid photocurable composition located at the positions is selectively (primarily) cured to form fabricated layers having the sectional shapes, and fabricated layers having sectional shapes at the heights are sequentially formed in accordance with the order of the serialization and stacked to obtain a stacked body having a shape corresponding to the shape of the three-dimensional object (hereinafter also referred to as "forming step"), and, as necessary, including a washing treatment with an organic solvent (hereinafter, this treatment step is also referred to as "washing step") and a secondary curing treatment (hereinafter, this treatment step is also referred to as "secondary curing step").

The curable composition for photofabrication according to the present disclosure characteristically contains: 100 parts by mass of radically-polymerizable monomer (A) that is liquid at 25°C; 50 to 250 parts by mass of inorganic filler (B); 0.05 to 5.0 parts by mass of photopolymerization initiator (C) that absorbs activating light to generate radicals; 0.01 to 2.5 parts by mass of activating light absorbent (D) that absorbs the activating light; 0.01 to 5.0 parts by mass of polymerization inhibitor (E); and 0.3 to 10 parts by mass of hyperbranched polymer (F) that is soluble in the radically-polymerizable monomer (A).

The above-described mass ratio of the radically-polymerizable monomer (A), the inorganic filler (B), and the hyperbranched polymer (F) makes it possible to enhance the strength and surface hardness of the three-dimensional photofabricated product. It is also possible to lower risks of increase in the composition viscosity and of inorganic filler particle sedimentation during storage. Furthermore, it is possible to prevent generation of visually-unrecognizable fine cracks on the surface of the three-dimensional photofabricated product.

Each of the components contained in the curable composition for photofabrication according to the present disclosure will be explained below. Each of the following components may be used alone or in combination of two or more types.

### [Radically-Polymerizable Monomer (A)]

As the radically-polymerizable monomer (A), any radically-polymerizable monomer that is liquid at 25°C can be used without particular limitation. Among radically-polymerizable monomers, (meth)acrylate type radically-polymerizable monomers are preferable in terms of their high curing rate and high strength of the resulting three-dimensional photofabricated product.

Furthermore, in terms of excellent strength of the resulting three-dimensional photofabricated product, it is preferable that 90 mass% or more (preferably 95 mass% or more, more preferably 100 mass%) of the radically-polymerizable monomer (A) is composed of a polyfunctional (meth)acrylate type radically-polymerizable monomer (a) having two or more (meth)acryloyl groups in its molecule and having 5 to 25 atoms constituting a main chain of a divalent group interposed between the two or more (meth)acryloyl groups present in its molecule. Herein, "5 to 25 atoms constituting a main chain of a divalent group" means that the number of atoms constituting the main chain of all "divalent groups" interposed between two adjacent (meth)acryloyl groups in the molecule is 5 to 25. For example, when the number of (meth)acryloyl groups in the molecule is 3, there will be two "divalent groups", and both the two "divalent groups" have the main chain composed of 5 to 25 atoms. Note that, when the main chain of the "divalent group" has a ring structure, specifically when the ring structure is introduced into the main chain via a bonding site in each of the two ring-constituting atoms, the number of the atoms constituting the main chain is counted with a pathway having fewer ring-constituting atoms between the two ring-constituting atoms as a part of the main chain.

When using the radically-polymerizable monomer (A) containing 90 mass% or more of such a polyfunctional (meth)acrylate type radically-polymerizable monomer (a), the partial decrease in the polymerization rate as described above can be suppressed and cracking can also be suppressed by a radical chain transfer resulting from addition of the chain transfer agent (G) described later.

Furthermore, the polyfunctional (meth)acrylate type radically-polymerizable monomer (a) is composed of a polyfunctional (meth)acrylate type radically-polymerizable monomer (a1) having at least one selected from a group consisting of a urethane bond (-NH-CO-O-), a urea bond (-NH-CO-NH-), and an isocyanurate ring, as well as another polyfunctional (meth)acrylate type radically-polymerizable monomer (a2). It is preferable that 40 to 90 mass% of the radically-polymerizable monomer (A) is the polyfunctional (meth)acrylate type radically-polymerizable monomer (a1), and 10 to 60 mass% is the polyfunctional (meth)acrylate type radically-polymerizable monomer (a2). A content of the polyfunctional (meth)acrylate type radically-polymerizable monomer (a1) in the radically-polymerizable monomer (A) is preferably 45 to 85 mass%, more preferably 48 to 82 mass%. A content of the polyfunctional (meth)acrylate type radically-polymerizable monomer (a2) in the radically-polymerizable monomer (A) is preferably 15 to 55 mass%, more preferably 18 to 52 mass%.

Examples of a compound suitable for use as the polyfunctional (meth)acrylate type radically-polymerizable monomer (a1) include a urethane group-containing (meth)acrylate such as 1,6-bis(methacryloyloxy-2-ethoxycarbonylamino)-2,2,4-trimethylhexane, 1,6-bis(methacryloyloxy-2-ethoxycarbonylamino)-2,4,4-trimethylhexane, phenylglycidyl ether-(meth)acrylate hexamethylene diisocyanate urethane prepolymer, pentaerythritol tri(meth)acrylate hexamethylene diisocyanate urethane prepolymer, pentaerythritol tri(meth)acrylate isophorone diisocyanate urethane prepolymer, and dipentaerythritol penta(meth)acrylate hexamethylene diisocyanate urethane prepolymer; an isocyanurate skeleton-containing meth(acrylate) such as tris(2-methacryloyloxyethyl) isocyanurate, caprolactone-modified tris-(2-acryloxyethyl) isocyanurate, and caprolactone-modified tris-(2-acryloxyethyl) isocyanurate. Above all, 1,6-bis(methacryloxy-2-ethoxycarbonylamino)-2,2,4-trimethylhexane, 1,6-bis(methacryloyloxy-2-ethoxycarbonylamino)-2,4,4-trimethylhexane, and caprolactone-modified tris-(2-acryloxyethyl) isocyanurate are preferable in terms of low viscosity and high strength.

Examples of a compound suitable for use as the polyfunctional (meth)acrylate type radically-polymerizable monomer (a2) include: a bisphenol A skeleton-containing (meth)acrylate such as 2,2'-bis{4-[3-(meth)acryloyloxy)-2-hydroxypropoxy]phenyl}propane, 2,2'-bis[4-(meth)acryloyloxyphenyl]propane, and 2,2'-bis[4-(meth)acryloyloxypolyethoxyphenyl]propane; an ethylene glycol-based (meth)acrylate such as triethylene glycol dimethacrylate and ethylene glycol dimethacrylate; an aliphatic di(meth)acrylate such as 1,3-propanediol di(meth)acrylate and 1,9-nonanediol dimethacrylate; and a trifunctional (meth)acrylate such as trimethylolpropane trimethacrylate. Above all, 2,2'-bis[4-(meth)acryloyloxyphenyl]propane, 2,2'-bis[4-(meth)acryloyloxy polyethoxyphenyl]propane, and triethylene glycol dimethacrylate are preferable in terms of low viscosity and high strength.

The radically-polymerizable monomer (A) may also contain a radically-polymerizable monomer other than the polyfunctional (meth)acrylate type radically-polymerizable monomer (a). Examples of another radically-polymerizable monomer include monofunctional (meth)acrylate type radically-polymerizable monomers such as hydroxyethyl (meth)acrylate, methyl (meth)acrylate, ethyl (meth)acrylate, isopropyl (meth)acrylate, tetrahydrofurfuryl (meth)acrylate, glycidyl (meth)acrylate, 2-methacryloxyethylpropionate, and acetoacetoxyethyl (meth)acrylate.

### [Inorganic Filler (B)]

To enhance the mechanical strength such as rigidity of the resulting three-dimensional photofabricated product and to prevent a decrease in the fluidity of the composition, the curable composition for photofabrication according to the present disclosure contains 50 to 250 parts by mass of inorganic filler (B) with respect to 100 parts by mass of the radically-polymerizable monomer (A). The inorganic filler (B) is composed of one or more types of inorganic powder granules. There is a tendency that, when the inorganic filler (B) content is excessively high, the curable composition for photofabrication has an excessively high viscosity. On the other hand, there is a tendency that, when the inorganic filler (B) content is excessively low, the obtained three-dimensional photofabricated product has an insufficient mechanical strength. For this reason, the inorganic filler (B) content is preferably 100 to 200 parts by mass with respect to 100 parts by mass of the radically-polymerizable monomer (A).

From the viewpoint of suppressing increase in the viscosity and particle sedimentation in the curable composition for photofabrication according to the present disclosure, it is preferable that, in the particle size distribution of the inorganic filler (B) measured by microscopy using a scanning microscope, 80% or more of all primary particles constituting the inorganic filler (B) have particle diameters of 0.05 to 5.0 µm. From the viewpoint of further suppressing increase in the viscosity of the curable composition for photofabrication, 80% or more of all primary particles constituting the inorganic filler (B) preferably have particle diameters of 0.08 µm or larger, more preferably 0.1 µm or larger. Furthermore, from the viewpoint of further suppressing particle sedimentation, it is more preferable that 80% or more of all primary particles constituting the inorganic filler (B) have particle diameters of 2.0 µm or smaller.

Herein, from the particle size distribution of the inorganic filler (B) measured by microscopy using a scanning microscope, it is possible to confirm that 80% or more of all primary particles constituting the inorganic filler (B) have particle diameters of 0.05 to 5.0 µm. Specifically, the inorganic filler (B) used for preparing the curable composition for photofabrication according to the present disclosure is photographed under a scanning electron microscope, then the number n (50 or more) of all primary particles observed in a unit visual field of the photograph is counted, and a primary particle diameter (maximum diameter): Xᵢ (nm) of each particle is measured for all primary particles to confirm a particle size distribution. Here, "i" in the above Xᵢ is a natural number of 1 to n, representing a number sign of each measured primary particle. The average (primary) particle diameter: X (nm) can be calculated according to an equation: X=ΣXᵢ/n, in which ΣXᵢ is a sum of measured particle diameters of all primary particles (i.e. primary particles with i=1 to n).

As the inorganic filler (B), for example, any inorganic powder granule that is usable as an inorganic filler for a dental restorative material can be used without particular limitation. Examples of a suitably usable inorganic powder granule include a powder granule composed of a metal (elemental); a powder granule composed of a metal oxide or a metal composite oxide; a powder granule composed of a metal fluoride, or a metal salt such as a carbonate, a sulfate, a silicate, a hydroxide, a chloride, a sulfite, and a phosphate; and a mixture of these powder granules. Examples of a particularly suitable inorganic filler constituent material include amorphous silica and quartz; a metal oxide such as alumina, zirconia, barium oxide, yttrium oxide, lanthanum oxide, and ytterbium oxide; a silica composite oxide such as silica-zirconia, silica-titania, silica-titania-barium oxide, and silica-titania-zirconia; glass such as borosilicate glass, aluminosilicate glass, and fluoroaluminosilicate glass; a metal fluoride such as barium fluoride, strontium fluoride, yttrium fluoride, lanthanum fluoride, and ytterbium fluoride; an inorganic carbonate such as calcium carbonate, magnesium carbonate, strontium carbonate, and barium carbonate; and a metal sulfate such as magnesium sulfate and barium sulfate. From the viewpoint of the wear resistance of the cured body, a silica-zirconia powder granule is most preferable. When producing dental restorations, a powder granule of silica-zirconia, silica-titania, silica-titania-barium oxide, silica-titania-zirconia, or the like is preferable, because of high X-ray contrasting property. From the viewpoint of the wear resistance of the cured body, a silica-zirconia powder granule is most preferable. For the inorganic filler (B), multiple types of powder granules made of different materials may be used in combination, or multiple types of powder granules having different particle diameters may be used in combination.

It is preferable that the inorganic filler (B) is treated with a surface treatment agent represented by a silane coupling agent in terms of improving compatibility with the polymerizable monomer and enhancing mechanical strength and water resistance. The surface treatment may be performed using any known method. Examples of the silane coupling agent include methyltrimethoxysilane, methyltriethoxysilane, methyltrichlorosilane, dimethyldichlorosilane, trimethylchlorosilane, vinyltrichlorosilane, vinyltriethoxysilane, vinyl tris(β-methoxyethoxy)silane, γ-methacryloyloxypropyl trimethoxysilane, methacryloxyoctyl-8-trimethoxysilane, γ-chloropropyltrimethoxysilane, γ-glycidoxypropylmethoxysilane, and hexamethyldisilazane.

### [Photopolymerization Initiator (C)]

The photopolymerization initiator (C) has a function of generating radicals via activating light containing light at a specific wavelength λ (nm) in the ultraviolet or visible light region, emitted from a light source mounted in a photofabrication apparatus, to cause radical polymerization of the polymerizable monomer. In other words, the photopolymerization initiator (C) needs to absorb light at a specific wavelength λ (nm) to generate radicals. The specific wavelength λ may be determined as appropriate depending on the wavelength of the activating light formed in the photofabrication apparatus as long as the specific wavelength λ is within the ultraviolet or visible light region. Examples of a general-purpose photofabrication apparatus include a stereo lithography apparatus (SLA) type photofabrication apparatus that emits a semiconductor laser light as the activating light, a digital light processing (DLP) type photofabrication apparatus that emits a projector light, and a liquid crystal display (LCD) type photofabrication apparatus that emits a liquid crystal panel light. Light sources with activating light at a wavelength of about 405 nm or about 385 nm are often used. Also in the present disclosure, as the specific wavelength λ, a wavelength of 405 nm or 385 nm is suitably adopted, and, as the photofabrication apparatus, an SLA type, DLP type, or LCD type apparatus is preferable.

The content of the photopolymerization initiator (C) should be 0.05 to 5.0 parts by mass with respect to 100 parts by mass of the radically-polymerizable monomer (A). If the content of the photopolymerization initiator (C) is excessively high, the resulting three-dimensional photofabricated product tends to have flash or the like, resulting in low shaping accuracy. On the other hand, if the content of the photopolymerization initiator (C) is excessively low, shaping in the forming step tends to become difficult. Thus, the content of the photopolymerization initiator is preferably 0.3 to 4.0 parts by mass, more preferably 0.5 to 3.0 parts by mass with respect to 100 parts by mass of the radically-polymerizable monomer (A).

As the photopolymerization initiator (C), any known photopolymerization initiator that satisfies the above conditions should be selected as appropriate for use. The photopolymerization initiator can be selected without particular limitation. Examples of the photopolymerization initiator include a self-cleavage photopolymerization initiator, a two-molecule hydrogen abstraction type photopolymerization initiator, a photoacid generator, and a combination thereof. These photopolymerization initiators may be used in combination with a photosensitizing dye, an electron-donating compound, or the like.

Examples of a suitable self-cleavage photopolymerization initiator include an acylphosphine oxide compound such as diphenyl(2,4,6-trimethylbenzoyl)-phosphine oxide and phenylbis(2,4,6-trimethylbenzoyl)-phosphine oxide; a benzoketal compound, a benzyne compound, an α-aminoacetophenone compound, an α-hydroxyacetophenone compound, a titanocene compound, and an acyloxime compound. Examples of a photoacid generator include an iodonium salt compound such as a p-isopropylphenyl-p-methylphenyl iodonium tetrakispentafluorophenyl borate salt; a sulfonium salt compound such as a dimethylphenacylsulfonium hexafluoroantimonate salt; and a halomethyl-substituted triazine compound such as 2,4,6-tris(trichloromethyl)-s-triazine. Examples of a photosensitizing dye include a ketone compound, a coumarin dye, a cyanine dye, a merocyanine dye, a thiazine dye, an azine dye, an acridine dye, a xanthene dye, a squarylium dye, a pyrylium salt dye, a condensed polycyclic aromatic compound (e.g. anthracene, perylene), and a thioxanthone compound. Examples of an electron-donating compound include 4-dimethylaminobenzoic acid ester and 4-dimethylaminotoluene, as well as p-dimethoxybenzene, 1,2,4-trimethoxybenzene, and a thiophene compound.

### [Activating Light Absorbent (D)]

For the purpose of preventing the activating light emitted from the photofabrication apparatus from being excessively transmitted and the shaping accuracy of the three-dimensional photofabricated product from being lowered, the curable composition for photofabrication according to the present disclosure contains the activating light absorbent (D) that absorbs the activating light emitted from the photofabrication apparatus and does not functionally serve as a photopolymerization initiator in an amount of 0.01 to 2.5 parts by mass with respect to 100 parts by mass of the radically-polymerizable monomer (A). If the content of the activating light absorbent (D) is excessively high, there is a tendency that the light emitted from the light source of the photofabrication apparatus does not transmit the curable composition for photofabrication in the forming step to make it difficult to produce the three-dimensional photofabricated product. On the other hand, if the content of the activating light absorbent (D) is excessively low, there is a tendency that the shaping accuracy of the resulting three-dimensional photofabricated product is lowered. For this reason, the content of the activating light absorbent (D) is preferably 0.04 to 2.5 parts by mass, more preferably 0.08 to 2.0 parts by mass, even more preferably 0.25 to 1.0 part by mass with respect to 100 parts by mass of the radically-polymerizable monomer (A).

The activating light absorbent (D) is not particularly limited as long as it is a compound that absorbs activating light emitted from a light source mounted in the photofabrication apparatus. Examples of the activating light absorbent (D) include a triazole compound such as 2-(hydroxy-5-methylphenyl)-2H-benzotriazole and 2-(3-tert-butyl-2-hydroxy-5-methylphenyl)-5-chloro-2H-benzotriazole; and a benzophenone compound such as 2,4-dihydroxybenzophenone and 2-hydroxy-4-methoxybenzophenone.

### [Polymerization Inhibitor (E)]

The curable composition for photofabrication according to the present disclosure contains 0.01 to 5.0 parts by mass of polymerization inhibitor (E) with respect to 100 parts by mass of the radically-polymerizable monomer (A) to improve its storage stability. If the content of the polymerization inhibitor (E) is excessively high, there is a tendency that the curable composition for photofabrication is insufficiently cured in the forming step. On the other hand, if the content of the polymerization inhibitor (E) is excessively low, there is a tendency that the storage stability of the curable composition for photofabrication is deteriorated, and the shaping accuracy of the resulting three-dimensional photofabricated product is lowered. For this reason, the content of the polymerization inhibitor (E) is preferably 0.01 to 5.0 parts by mass, more preferably 0.03 to 4.0 parts by mass, even more preferably 0.05 to 2.5 parts by mass.

As the polymerization inhibitor (E), a compound that reacts with radicals generated in the curable composition for photofabrication to deactivate the radicals can be used. Examples of the polymerization inhibitor (E) include di-tert-butyl-p-cresol and 4-methoxyphenol.

### [Hyperbranched Polymer (F)]

The hyperbranched polymer is a multibranched polymer obtained by a single-step polymerization of a polyfunctional monomer having two or more types of functional groups, having branching points in its repeating units, and a three-dimensionally expanded molecular chain.

As described in Patent Document 5, a hyperbranched polymer is blended in a dental curable composition for use in a dental composite resin to reduce polymerization shrinkage of the composition. In the present disclosure, such a hyperbranched polymer that is soluble in the radically-polymerizable monomer (A) can be used without particular limitation.

Herein, "soluble in the radically-polymerizable monomer (A)" means that the solubility in the radically-polymerizable monomer (A) at 25°C {limit amount (g) of the hyperbranched polymer (F) soluble in 100 g of the radically-polymerizable monomer (A)} is 5 (g) or higher, preferably 10 (g) or higher. Thus, in the curable composition for photofabrication, the blended hyperbranched polymer (F) only needs to be partially dissolved, and need not be thoroughly dissolved. However, it is preferable that the hyperbranched polymer (F) is thoroughly dissolved. In other words, it is preferable that the hyperbranched polymer (F) has a solubility sufficient to thoroughly dissolve in the radically-polymerizable monomer (A).

In the present disclosure, simply, 5 g or 10 g of the hyperbranched polymer (F) is added to 100 g of the radically-polymerizable monomer (A), and it is visually confirmed whether or not the hyperbranched polymer (F) dissolves in the radically-polymerizable monomer (A) at 25°C. For example, if 5 g of the hyperbranched polymer (F) does not thoroughly dissolve, the solubility is lower than 5 (g), and if 5 g of the hyperbranched polymer (F) thoroughly dissolves, the solubility is 5 (g) or higher.

The radically-polymerizable monomer (A) may be used in combination of two or more polymerizable monomers. When the radically-polymerizable monomer (A) is used in combination of two or more polymerizable monomers, the solubility of the hyperbranched polymer (F) in the radically-polymerizable monomer (A) only needs to be 5 (g) or higher in the mixture of two or more polymerizable monomers as the radically-polymerizable monomer (A). The polymerizable monomers constituting the mixture may contain polymerizable monomers with a hyperbranched polymer (F) solubility of lower than 5 (g).

The hyperbranched polymer (F) preferably includes a first unit structure having four bonding sites and represented by the following formula (I) and a second unit structure that is at least one of two unit structures represented by the following formula (IIA) and the following formula (IIB) (hereinafter also referred to as "specific hyperbranched polymer"). 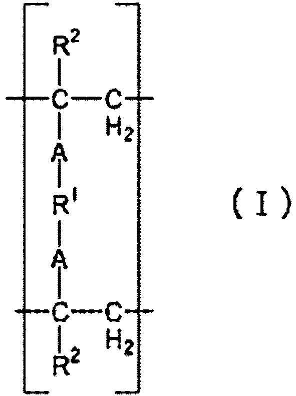 In the above formula (I), A each independently represent a single bond for bonding C and R¹, >C=O, -O-, -COO-, or -COO-CH₂-, R¹ represents a divalent saturated aliphatic hydrocarbon group or a divalent aromatic hydrocarbon group, and R² each independently represent hydrogen atom or a methyl group. 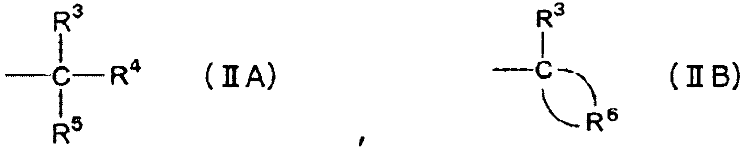 In the above formulas (IIA) and (IIB), R³, R⁴, and R⁵ each independently represent hydrogen atom, an alkyl group having 1 to 5 carbon atoms for forming a main chain, an alkoxycarbonyl group having 2 to 6 carbon atoms for forming a main chain, an aryl group, or a cyano group, and R⁶ represents an alkylene group having 4 to 10 carbon atoms for forming a main chain.

The first unit structure having four bonding sites and represented by the above formula (I) forms a multibranched structure of a specific hyperbranched polymer. The first unit structure represented by the above formula (I) or the second unit structure that is at least one of two unit structures represented by the above formulas (IIA) and (IIB) can bind to the four bonding sites. Herein, the first unit structures bind to each other via at least one of the four bonding sites to form a multibranched structure. The second unit structure as a terminal group that divides the multibranched structure can bind to at most three of the four bonding sites of the first unit structure.

A content ratio (molar ratio) of the first unit structure and the second unit structure contained in the specific hyperbranched polymer is preferably 3:7 to 7:3, more preferably 4:6 to 6:4. When the molar ratio is within the above range, a multibranched structure with moderate branches can be formed, and furthermore, extreme increase in a viscosity of a solution with the specific hyperbranched polymer dissolved in a solvent can be suppressed.

R¹ in the above formula (I) represents a divalent saturated aliphatic hydrocarbon group or a divalent aromatic hydrocarbon group.

The divalent saturated aliphatic hydrocarbon group may be either acyclic or cyclic. The number of carbon atoms in the divalent saturated aliphatic hydrocarbon group is preferably 1 to 5, more preferably 1 or 2, without particular limitation. When the number of carbon atoms is 5 or less, the molecular chain portion represented by R¹ becomes shorter, and therefore entanglement between the radically-polymerizable monomer (A) and the hyperbranched polymer (F) can be suppressed, resulting in suppression of an increase in viscosity of the curable composition for photofabrication. Examples of the divalent acyclic saturated aliphatic hydrocarbon group include a methylene group, an ethylene group, a propylene group, and a butylene group. Examples of the divalent cyclic saturated aliphatic hydrocarbon group include a cyclopropylene group and a cyclobutylene group.

The divalent aromatic hydrocarbon group may be any of a monocyclic group containing one benzene ring, a group having a condensed ring structure containing two or more benzene rings, and a group having no condensed ring structure containing two or more benzene rings. The number of benzene rings contained in the divalent aromatic hydrocarbon group is preferably 1 to 2 without particular limitation. When the number of the benzene rings is 2 or less, entanglement between the radically-polymerizable monomer (A) and the hyperbranched polymer (F) can be suppressed, resulting in suppression of an increase in viscosity of the curable composition for photofabrication. Examples of the divalent aromatic hydrocarbon group include a phenylene group, a naphthylene group, and a biphenylene group.

R³, R⁴, and R⁵ in the above formulas (IIA) and (IIB) each independently represent hydrogen atom, an alkyl group having 1 to 5 carbon atoms for forming a main chain, an alkoxycarbonyl group having 2 to 6 carbon atoms for forming a main chain, an aryl group, or a cyano group.

Examples of the alkyl group include a methyl group, an ethyl group, and a propyl group. Examples of the alkoxycarbonyl group include a methoxycarbonyl group, an ethoxycarbonyl group, and a propoxycarbonyl group. Examples of the aryl group include a phenyl group. As for the alkyl group and the alkoxycarbonyl group, when the number of carbon atoms for forming a main chain is 5 or less, in particular, when a methyl group or a methoxycarbonyl group is adopted, their main chains become shorter, and therefore entanglement between the radically-polymerizable monomer (A) and the hyperbranched polymer (F) can be suppressed, resulting in suppression of an increase in viscosity of the curable composition for photofabrication.

Some hydrogen atoms in the alkyl group, the alkoxycarbonyl group, and the aryl group may be substituted. The substituent is not particularly limited unless the substituent has a coloring structure or group (e.g. a reactive unsaturated bond, an amino group, a hydroxy group). Examples of the substituent include an alkyl group (e.g. a methyl group) having 1 to 3 carbon atoms and an alkoxy group (e.g. a methoxy group) having 1 to 3 carbon atoms. When the alkyl group and the alkoxy group is a group having 3 or less carbon atoms, particularly a methyl group or a methoxy group, their main chains become shorter, and therefore entanglement between the radically-polymerizable monomer (A) and the hyperbranched polymer (F) can be suppressed, resulting in suppression of an increase in viscosity of the curable composition for photofabrication.

In the above formula (IIB), R⁶ represents an alkylene group having 4 to 10 carbon atoms for forming a main chain.

Examples of the alkylene group include a butylene group, a pentylene group, and a nonylene group. Some hydrogen atoms in the alkylene group may be substituted. The substituent is not particularly limited unless the substituent has a coloring structure or group (e.g. a reactive unsaturated bond, an amino group, a hydroxy group). Examples of the substituent include an alkyl group (e.g. a methyl group) having 1 to 3 carbon atoms and an alkoxy group (e.g. a methoxy group) having 1 to 3 carbon atoms. When the main chain of the alkylene group has 4 or more carbon atoms, such a risk that the ring containing the group R⁶ becomes unstable to react with surrounding substances, resulting in coloring can be suppressed. Furthermore, when the main chain of the alkylene group has 10 or less carbon atoms, or when the alkyl group and the alkoxy group selected as the substituent is a group having 3 or less carbon atoms, particularly a methyl group or a methoxy group, their main chains become shorter, and therefore entanglement between the radically-polymerizable monomer (A) and the hyperbranched polymer (F) can be suppressed, resulting in suppression of an increase in viscosity of the curable composition for photofabrication.

The second unit structure represented by the above formulas (IIA) and (IIB) is originated from raw material components usable in a synthesis process of a specific hyperbranched polymer. Examples of the raw material components include a known polymerization initiator, preferably an azo polymerization initiator disclosed in PCT International Publication No. WO 2010/126140. However, after synthesis of the specific hyperbranched polymer is completed, it is possible to adopt only a hyperbranched polymer capable of having a structure represented by the above formula (IIA) or (IIB).

Specific examples of the second unit structure represented by the above formula (IIA) include the following structural formulas A to K. Specific examples of the second unit structure represented by the above formula (IIB) include the following structural formulas L and M. The second unit structures represented by these structural formulas A to M are particularly suitable in terms of availability of the specific hyperbranched polymer.

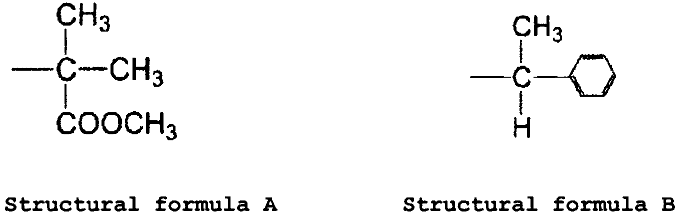

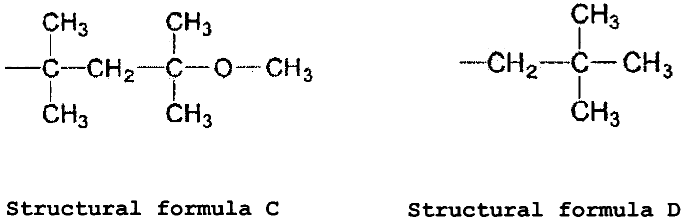

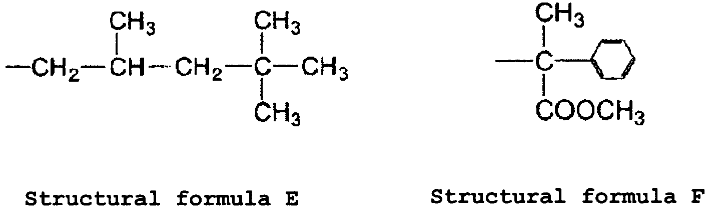

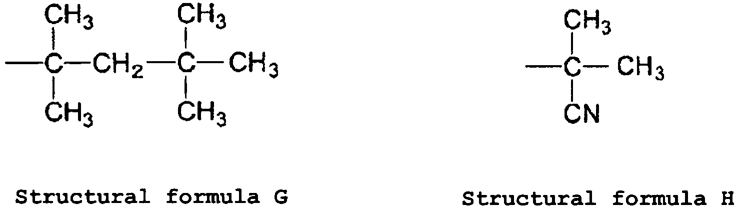

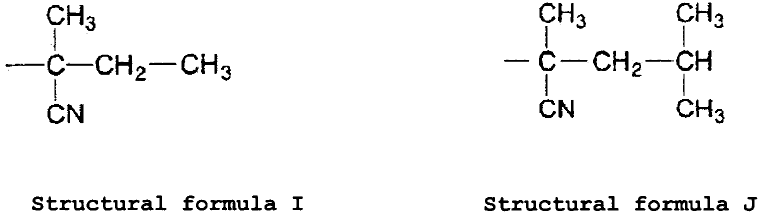

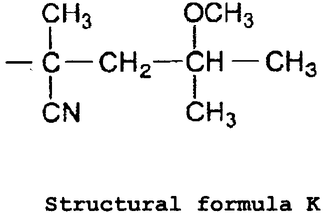

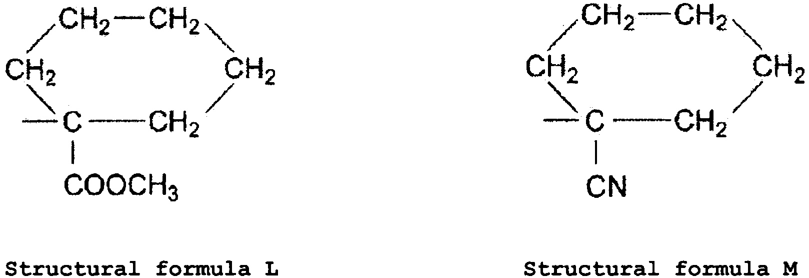

Preferably, the specific hyperbranched polymer has a mass-average molecular weight of 1,000 to 200,000. This mass-average molecular weight is a polystyrene-equivalent value determined by a gel permeation chromatography (GPC) using tetrahydrofuran as a solvent. Since the specific hyperbranched polymer is soluble in the radically-polymerizable monomer (A) and available as a powder granule composed of spherical particles having a hydrodynamic average diameter of about 5 to 40 nm, which can be easily dispersed uniformly, the specific hyperbranched polymer has a mass-average molecular weight of more preferably 5,000 to 100,000, even more preferably 20,000 to 50,000. The hydrodynamic average diameter of the specific hyperbranched polymer is measured in tetrahydrofuran at 40°C by a dynamic light scattering method. The specific hyperbranched polymer has a hydrodynamic average diameter of preferably 5 to 20 nm, more preferably 5 to 15 nm.

The hyperbranched polymer composed of such a powder granule is industrially available, and for example, "HYPERTECH" (registered trademark) manufactured by Nissan Chemical Corporation, Ltd., "HYBRANE" (registered trademark) manufactured by DSM N.V., and "BOLTORN" (registered trademark) manufactured by Perstorp AB are commercially available.

The content of the hyperbranched polymer (F) only needs to be 0.3 to 10 parts by mass with respect to 100 parts by mass of the radically-polymerizable monomer (A). If the content of the hyperbranched polymer (F) is too high, the viscosity of the curable composition for photofabrication tends to increase. On the other hand, if the content of the hyperbranched polymer (F) is excessively low, the cracking prevention effect tends to be insufficient. For this reason, the content of the hyperbranched polymer (F) is preferably 2 to 10 parts by mass, more preferably 2 to 8 parts by mass, even more preferably 4 to 6 parts by mass, with respect to 100 parts by mass of the radically-polymerizable monomer (A).

### [Chain Transfer Agent (G)]

For the purpose of reducing the shrinkage stress during the photofabrication and improving the shaping accuracy of the three-dimensional photofabricated product, the curable composition for photofabrication according to the present disclosure may contain a chain transfer agent in an amount of 0.005 to 1.0 part by mass with respect to 100 parts by mass of the radically-polymerizable monomer (A). When the radically-polymerizable monomer (A) contains 90 mass% or more of polyfunctional (meth)acrylate type radically-polymerizable monomer (a), it is preferable that the composition contains the chain transfer agent (G) in an amount of 0.01 to 1.0 part by mass, preferably 0.03 to 0.3 part by mass with respect to 100 parts by mass of radically-polymerizable monomer (A) because the cracking prevention effect can be improved.

Examples of the chain transfer agent (G) include a thiol compound such as butanethiol, thiophenol, mercaptoethanol, octylthiol, and laurylmercaptan; an α-alkylstyrene compound such as 2,4-diphenyl-4-methyl-1-pentene (α-methylstyrene dimer), and 2-phenyl-1-propene (α-methylstyrene); and a halogenated hydrocarbon substituted with at least one halogen atom, such as carbon tetrachloride and ethylene bromide. Above all, an α-alkylstyrene compound is preferable and an α-methylstyrene dimer is more preferable, because of a high cracking suppression effect.

### [Other Components]

### (Thermal Polymerization Initiator)

The curable composition for photofabrication according to the present disclosure may contain a thermal polymerization initiator as a polymerization initiator for the secondary curing. In this case, it is preferable to use a thermal polymerization initiator with a 10-hour half-life temperature of 50 to 130°C, because the thermal polymerization initiator does not function during the primary curing but effectively remains in the stacked body. Examples of a suitably usable thermal polymerization initiator include an organic peroxide such as tert-butylperoxylaurate and benzoyl peroxide; and an azo compound such as azobutyronitrile and azobis(dimethylvaleronitrile).

When the curable composition for photofabrication according to the present disclosure contains a thermal polymerization initiator, a content of the thermal polymerization initiator is typically 0.001 to 1.0 part by mass, preferably 0.005 to 0.3 part by mass, more preferably 0.01 to 0.1 part by mass, with respect to 100 parts by mass of the radically-polymerizable monomer (A).

### (Coloring Substance)

The curable composition for photofabrication according to the present disclosure may contain a coloring substance unless the effects of the present invention are not inhibited. For example, when the curable composition for photofabrication according to the present disclosure is used to produce a dental restoration such as an inlay, an onlay, a crown, and a denture, it is preferable to blend a coloring substance in the composition to reproduce a crown color or an oral mucosa color. The coloring substance may be a pigment or a dye. Examples of the pigment include an inorganic pigment such as titanium oxide, zinc oxide, zirconium oxide, zinc sulfide, aluminum silicate, calcium silicate, carbon black, iron oxide, copper chromite black, chromium oxide green, chrome green, violet, chrome yellow, lead chromate, lead molybdate, cadmium titanate, nickel-titanium yellow, ultramarine blue, cobalt blue, bismuth vanadate, cadmium yellow, and cadmium yellow; an organic pigment such as monoazo pigment, diazo pigment, diazo condensation pigment, perylene pigment, and anthraquinone pigment.

### <Method for Producing Curable Composition for Photofabrication>

To produce the curable composition for photofabrication according to the present disclosure, the aforementioned components are mixed to achieve a predetermined composition. In terms of stable production of the composition that reliably exhibits the effects, it is preferable to adopt a method in which the radically-polymerizable monomer (A), the photopolymerization initiator (C), the activating light absorbent (D), the polymerization inhibitor (E), and the hyperbranched polymer (F) are mixed to prepare a liquid composition into which at least a part of the hyperbranched polymer is dissolved, and then the obtained liquid composition is mixed with the inorganic filler (B). When preparing the liquid composition, it is preferable that a powder granule of the hyperbranched polymer having a hydrodynamic average diameter of 2 to 40 nm is mixed in the liquid composition.

It is preferable that the components are mixed using a stirrer while shielding a light that activates the photopolymerization initiator (C), e.g. under red light, at room temperature or under warming at 40 to 80°C until a uniform mixture is obtained. After mixing of the inorganic filler (B), it is preferable to perform a defoaming treatment.

When using the hyperbranched polymer (F) that is thoroughly soluble in the radically-polymerizable monomer (A), it is preferable to adopt a method in which the mixing is performed at 40 to 80°C during preparation of the liquid composition, or a method in which the hyperbranched polymer (F) is dissolved and mixed in a volatile organic solvent, and then the organic solvent is removed to prepare the liquid composition, since it becomes possible to reliably produce a curable composition for photofabrication in which the hyperbranched polymer (F) is thoroughly dissolved. Examples of the volatile organic solvent used in the latter method include acetone, methylethylketone, acetonitrile, tetrahydrofuran, diethyl ether, pentane, hexane, ethyl acetate, methanol, ethanol, 1-propanol, isopropanol, water, chloroform, dichloromethane, and trichloroethane. Each of these volatile organic solvents may be used alone, or, if they can be uniformly mixed, may be used in combination of two or more types. As the volatile organic solvent removal method, a known method such as heating and decompression can be adopted.

### <Method for Producing Three-Dimensional Photofabricated Product>

A three-dimensional photofabricated product can be produced from the curable composition for photofabrication according to the present disclosure using a vat photopolymerization method. Specifically, a desired three-dimensional photofabricated product can be produced by sequentially performing a forming step that, from three-dimensional shape data indicating a shape of a three-dimensional object, a height direction of the three-dimensional object is digitized and serialized, and two-dimensional shape data indicating sectional shapes of the three-dimensional object at the serialized heights is generated, and then, using a vat photopolymerization method including irradiating a predetermined position of a liquid curable composition for photofabrication according to the present disclosure in a vat with activating light that is ultraviolet ray or visual light to selectively cure the curable composition for photofabrication at the position, fabricated layers having shapes corresponding to the two-dimensional shapes at the heights are sequentially formed and stacked in accordance with the order of the serialization on the basis of the two-dimensional shape data, to obtain a stacked body having a shape corresponding to the three-dimensional object shape; a washing step that the stacked body obtained in the forming step is washed with an organic solvent; and a secondary curing step that the washed stacked body is secondarily cured. According to this production method, a three-dimensional photofabricated product having a high mechanical strength and no cracks on its actual surface can be produced.

In the method for producing the three-dimensional photofabricated product according to the present disclosure, it is preferable that the forming step includes:
a first step that, based on the two-dimensional shape data at the heights in the initial serialization order, a predetermined position of the curable composition for photofabrication in the vat is irradiated with activating light to cure the composition, thereby forming a fabricated layer having a shape corresponding to the two-dimensional shape data, so that the shaped layer serves as a layer for bonding;
a second step that the layer for bonding is moved up and down to feed the curable composition for photofabrication to a position directly above or below the aforementioned layer for bonding in the vat;
a third step that, based on the two-dimensional shape data at heights of a serialization order following the serialization order in the previous step, a predetermined position of the curable composition for photofabrication fed to the position directly above or below the layer for bonding is irradiated with activating light to cure the composition to form a new fabricated layer having a shape corresponding to the two-dimensional data, and this fabricated layer is bonded to the layer for bonding to obtain a stacked body having the new fabricated layer as a new layer for bonding; and
a fourth step that the stacked body is moved up and down to feed the curable composition for photofabrication to a position directly above or below the aforementioned new layer for bonding in the vat, in which
using the new layer for bonding as the layer for bonding in the third step, a cycle of the third and fourth steps is repeated, and in the final third step, a new fabricated layer is formed based on the two-dimensional shape data at heights in the final serialization order, to obtain a stacked body.

The vat photopolymerization method including such a forming step can be suitably performed using a commercially available vat photopolymerization apparatus referred to as a so-called 3D printer.

Examples of the organic solvent in the washing step include an alcohol solvent such as ethanol, methanol, and isopropyl alcohol; a ketone solvent such as acetone and methylethylketone; an ether solvent such as diethyl ether, diisopropyl ether, tripropylene glycol monomethyl ether, and tetrahydrofuran; an amide solvent such as N-methylpyrrolidone and dimethylacetamide; a halogen solvent such as methylene chloride and chloroform. Above all, an alcohol solvent and an ether solvent are preferable because of a high washing efficiency, and an alcohol solvent is more preferable because of a low environmental load.

In the secondary curing step, secondary curing is performed by additional activating light irradiation, heating treatment, or both of them. When performing additional light irradiation, the activating light irradiation wavelength is not particularly limited as long as, at the wavelength, the photopolymerization initiator remaining in the stacked body absorbs the light to generate radicals. The irradiation intensity for the additional light irradiation is preferably 5 mW/cm² or higher, more preferably 10 mW/cm² or higher, even more preferably 30 mW/cm² or higher for the photopolymerization initiator remaining in the stacked body to generate a sufficient quantity of radicals. The irradiation time is preferably 1 minute or longer, more preferably 3 minutes or longer, even more preferably 5 minutes or longer without particular limitation. If the irradiation intensity during the additional light irradiation is excessively high, the three-dimensional photofabricated product is excessively heated to cause cracks in the three-dimensional photofabricated product in some cases. For this reason, the irradiation intensity is preferably 10,000 mW/cm² or lower.

When the curable composition for photofabrication according to the present disclosure contains a thermal polymerization initiator, the composition can be secondarily cured by heating using this thermal polymerization initiator. In this process, the heating temperature is preferably 45 to 120°C, more preferably 50 to 90°C, even more preferably 55 to 80°C.

Also, the method for producing the three-dimensional photofabricated product according to the present disclosure makes it possible to produce a dental restoration such as an inlay, an onlay, a crown, and a denture. When producing such a dental restoration, CAD data should be used, which is designed based on digital data obtained by scanning an intraoral shape of an individual patient or an intraoral model prepared for each individual patient as three-dimensional shape data indicating a shape of a dental restoration (three-dimensional object) for use in the forming step. This production method makes it possible to produce a dental restoration having a high mechanical strength and free from cracks on its actual surface.

### EXAMPLES

The present invention will be explained below in more detail with reference to Examples, but the present invention is not limited to these Examples.

The names, properties, and abbreviations (if present) of the various compounds used in Examples and Comparative Examples will be described below.

### <(A) Radically-Polymerizable Monomer>

[Polyfunctional (meth)acrylate type radically-polymerizable monomer (polyfunctional monomer) (a1)]
   - UDMA: Urethane dimethacrylate
   - A-9300-1CL: Caprolactone-modified tris-(2-acryloxyethyl) isocyanurate (manufactured by SHIN-NAKAMURA CHEMICAL CO, LTD.) [Polyfunctional (meth)acrylate type radically-polymerizable monomer (polyfunctional monomer) (a2)]
   - 3G: Triethylene glycol dimethacrylate
   - D-2.6E: Bisphenol A ethylene glycol (EO) adduct dimethacrylate (EO adduct number: 2.6 on average)
[Other radicallypolymerizable monomers (other monomers)]
   - 9G: Nonaethylene glycol dimethacrylate
   - 1G: Monoethylene glycol dimethacrylate
   - ACMO: Acryloyl morpholine

### <(B) Inorganic Filler>

- Composition A: Filler containing a mixture of 70 parts by mass of filler B1 that is a spherical silica-zirconia powder granule (with a surface treated with γ-methacryloyloxypropyl trimethoxysilane) having an average primary particle diameter of 0.5 µm, and 30 parts by mass of filler B2 that is a spherical silica-zirconia powder granule (with a surface treated with γ-methacryloyloxypropyl trimethoxysilane) having an average primary particle diameter of 0.08 µm.
- Composition B: Filler containing a mixture of 70 parts by mass of filler B1 that is a spherical silica-zirconia powder granule (with a surface treated with γ-methacryloyloxypropyl trimethoxysilane) having an average primary particle diameter of 0.5 µm, and 30 parts by mass of filler B3 that is a spherical silica-titania powder granule (with a surface treated with γ-methacryloyloxypropyl trimethoxysilane) having an average primary particle diameter of 0.3 µm.
- Composition C: Spherical silica powder granule (with a surface treated with γ-methacryloyloxypropyl trimethoxysilane) having an average primary particle diameter of 1000 nm

### <(C) Photopolymerization Initiator>

- BTPO: Phenylbis(2,4,6-trimethylbenzoyl)-phosphine oxide
- CQ: Camphorquinone
- TPO: 2,4,6-Trimethylbenzoyl-diphenylphosphine oxide

### <(D) Activating Light Absorbent>

- SS3: 2-(3-tert-butyl-2-hydroxy-5-methylphenyl)-5-chloro-2H-benzotriazole

### <(E) Polymerization Inhibitor>

- HQME: Hydroquinone methyl ether
- BHT: Dibutylhydroxytoluene

### <(F) Hyperbranched Polymer>

- HBPA: Poly(ethylene glycol dimethacrylate-methyl isobutyrate) (HYPERTEC HA-DMA-200, manufactured by Nissan Chemical Corporation, mass-average molecular weight: 22,000, hydrodynamic average diameter: 5.2 nm, represented by the following structural formula)

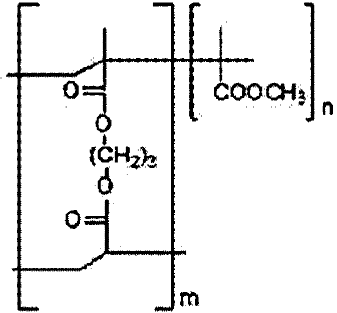

- HBPB: Poly(divinylbenzene-methyl isobutyrate) (HYPERTEC HA-DVB-500, manufactured by Nissan Chemical Corporation, mass-average molecular weight: 48,000, hydrodynamic average diameter: 11.7 nm, represented by the following structural formula)

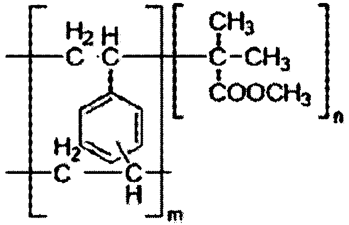

### <(G) Chain Transfer Agent>

- MSD: α-methylstyrene dimer

### <Other Components>

- PMMA: Polymethyl methacrylate (mass-average molecular weight: 250,000, average particle diameter: 30 µm)
- DMBE: Ethyl p-dimethylaminobenzoate ester

The evaluation methods for various physical properties in Examples and Comparative Examples are as follows.

### (1) Method for Measuring Solubility of Hyperbranched Polymer

Each of the hyperbranched polymers was added in 10 g of the radically-polymerizable monomer mixture in each of Examples and Comparative examples, and the solubility was measured by visually confirming whether the hyperbranched polymer was dissolved in the mixture at 25°C. The notations in the column "Solubility" in Tables 2 and 4 presented later have the following meanings:
<5: the solubility is lower than 5 (g) (0.5 g of the hyperbranched polymer is not thoroughly dissolved in 10 g of the monomer)
≥10: the solubility is 10 (g) or higher (1 g of the hyperbranched polymer is thoroughly dissolved in 10 g of the monomer)

### (2) Method for Evaluating Curable Composition for Photofabrication

### [Method for Measuring Viscosity]

On a sample stage of a rheometer MCR302 (manufactured by Anton Paar GmbH), 0.7 g of the curable composition for photofabrication in each of Examples and Comparative Examples was placed, and the paste was pressed between parallel plates having a diameter of 20 mm such that the paste had a thickness of 1 mm. Subsequently, the paste was rotated at a shear rate of 5 (s⁻¹) for 300 seconds, and its viscosity at a time point of 300 seconds was measured three times. The average of the three viscosities was rounded to the nearest hundred, and the rounded value was defined as a viscosity of the curable composition for photofabrication in each of Examples and Comparative Examples.

### [Method for Evaluating Inorganic Filler Sedimentation]

The curable composition for photofabrication in each of Examples and Comparative Examples was taken out in an amount of 10 g, then charged into a cylindrical light-shielding container having a diameter of 50 mm and a height of 28 mm, and stored in a thermostat at 25°C. After three months, it was visually confirmed whether a sediment layer had formed at the bottom of the cylindrical light-shielding container.

### (3) Method for Producing Three-Dimensional Photofabricated Product

The curable composition for photofabrication in each of Examples and Comparative Examples was fed to a resin tray (tank) of a 3D printer (DW029D manufactured by DWS Company, wavelength: 405 nm, irradiation intensity: 83 mW) to prepare a formed body (stacked body) having a stacked structure having a rectangular parallelepiped shape of 10 mm×10 mm×25 mm and based on stereolithography data (hereinafter abbreviated as "stl data") (composed of a cured body of the curable composition for photofabrication). Then, the obtained formed body was immersed in ethanol in a plastic container for 15 minutes, then washed under moderate vibration, then dried, and further irradiated with light using a UV Curing Unit UVIS-2 (manufactured by DWS Company) for 30 minutes (secondary curing) to produce a three-dimensional photofabricated product.

### (4) Method for Evaluating Three-Dimensional Photofabricated Product

### [Method for Measuring Bending Strength]

After performing the cracking evaluation described below, the evaluation sample was abraded using #800 waterproof abrasive paper and shaped into a prism shape of 2 mm×2 mm×25 mm. This test piece was attached to a testing machine (AUTOGRAPH AG5000D manufactured by Shimadzu Corporation), then its 3-point bending fracture strength was measured five times with an inter-fulcrum distance of 20 mm and a crosshead speed of 1 mm/min, and an arithmetic average value of the five 3-point bending fracture strengths was calculated.

### [Method for Evaluating Cracking]

The surface of the evaluation sample was observed using an optical microscope (magnification: 50X), then the evaluation sample was coated with platinum in a thickness of 5 nm, and observed using a scanning microscope (magnification: 1000X). The number and widths of cracks observed on one 10 mm×25 mm face of the evaluation specimen were confirmed and evaluated in accordance with the following evaluation criteria.

### -Evaluation Criteria-

A1: No more than 5 cracks are observed on the surface of the cured body, and all cracks have a width of smaller than 10 µm, within the acceptable range.

A2: No more than 10 cracks are observed on the surface of the cured body, and all cracks have a width of smaller than 10 µm, within the acceptable range.

A3: No more than 20 cracks are observed on the surface of the cured body, and all cracks have a width of smaller than 10 µm, within the acceptable range.

B: No more than 20 cracks are observed on the surface of the cured body, and all cracks have a width of 10 to 40 µm.

C: No less than 20 cracks having a width of 10 to 40 µm are observed on the surface of the cured body.

### <Example 1>

UDMA (80 parts by mass), 9G (20 parts by mass), BTPO (1.4 part by mass), SS3 (0.7 part by mass), HQME (0.1 part by mass), and BHT (0.1 part by mass) were stirred and mixed until a uniform mixture was obtained, to which HBPA (5 parts by mass) was added and further stirred. The absence of undissolved components was visually confirmed. The solubility of HBPA in the mixture of UDMA and 9G was 10 (g) or higher. An inorganic filler composition A (150 parts by mass) was added to the resulting mixture, and stirred and mixed until a uniform mixture was obtained. The mixture was defoamed to prepare a liquid curable composition for photofabrication.

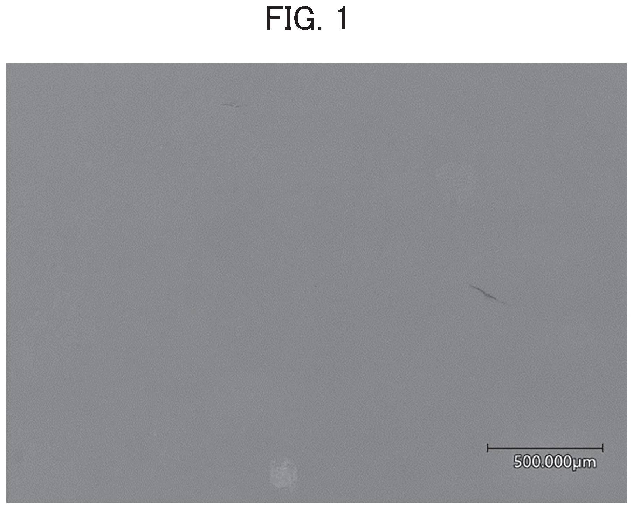

The viscosity of the resulting curable composition for photofabrication was measured, and the inorganic filler sedimentation was evaluated. As a result, the viscosity was 7000 mPa·s with no sedimentation. A three-dimensional photofabricated product was prepared from the obtained curable composition for photofabrication, and a bending strength and cracks of the article were evaluated. As a result, the bending strength was 121 MPa, and the cracking evaluation result was rated as "A3". FIG. 1 presents an optical microscope image (magnification: 50X) photographed during the observation in the cracking evaluation of Example 1.

### <Examples 2, 5 to 19, 21 to 23>

A curable composition for photofabrication was prepared in the same manner as in Example 1, except that the composition of the curable composition for photofabrication was changed as presented in Tables 1 and 2 below. The parenthetical numerical values in Tables 1 and 2 indicate the blending amounts of each component (unit: parts by mass). Furthermore, a three-dimensional photofabricated product was prepared from the obtained curable composition for photofabrication. For the curable composition for photofabrication and the three-dimensional photofabricated product, the same evaluation as in Example 1 was performed. The results are presented in Table 4 below.

### <Example 3>

HBPA (10 parts by mass) was added to acetone, which was stirred and mixed. The absence of undissolved components was visually confirmed. UDMA (80 parts by mass), 3G (20 parts by mass), BTPO (1.4 part by mass), SS3 (0.7 part by mass), HQME (0.1 parts by mass), and BHT (0.1 part by mass) were stirred and mixed until a uniform mixture was obtained, to which the acetone solution of HBPA was added, which was further stirred and mixed. Uniformity of the mixture was confirmed, and acetone was removed from the mixture using a rotary evaporator. Subsequently, an inorganic filler composition A (150 parts by mass) was added to the obtained mixture, and stirred and mixed until a uniform mixture was obtained. The mixture was defoamed to prepare a liquid curable composition for photofabrication. Furthermore, a three-dimensional photofabricated product was prepared from the obtained curable composition for photofabrication. For the curable composition for photofabrication and the three-dimensional photofabricated product, the same evaluation as in Example 1 was performed. The results are presented in Table 4 below.

### <Example 4>

UDMA (80 parts by mass), 3G (20 parts by mass), BTPO (1.4 part by mass), SS3 (0.7 part by mass), HQME (0.1 part by mass), and BHT (0.1 part by mass) were stirred and mixed until a uniform mixture was obtained, to which HBPA (7 parts by mass) was added, and stirred and mixed while heating at 45°C. The absence of undissolved components was visually confirmed. An inorganic filler composition A (150 parts by mass) was added to the resulting mixture, and stirred and mixed until a uniform mixture was obtained. The mixture was defoamed to prepare a liquid curable composition for photofabrication. Furthermore, a three-dimensional photofabricated product was prepared from the obtained curable composition for photofabrication. For the curable composition for photofabrication and the three-dimensional photofabricated product, the same evaluation as in Example 1 was performed. The results are presented in Table 4 below.

### <Example 20>

UDMA (80 parts by mass), 3G (20 parts by mass), BTPO (1.4 part by mass), SS3 (0.7 part by mass), HQME (0.1 part by mass), and BHT (0.1 part by mass) were stirred and mixed until a uniform mixture was obtained. HBPA (5 parts by mass) and the inorganic filler composition A (150 parts by mass) were added to the resulting mixture, and stirred and mixed until a uniform mixture was obtained. The mixture was defoamed to prepare a liquid curable composition for photofabrication. Furthermore, a three-dimensional photofabricated product was prepared from the obtained curable composition for photofabrication. For the curable composition for photofabrication and the three-dimensional photofabricated product, the same evaluation as in Example 1 was performed. The results are presented in Table 4 below.

### <Comparative Examples 1 and 2>

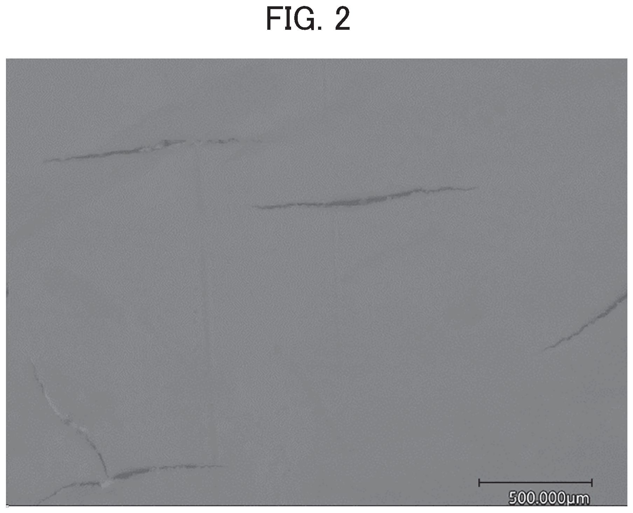

A curable composition for photofabrication was prepared in the same manner as in Example 1, except that the composition of the curable composition for photofabrication was changed as presented in Table 3 below. The parenthetical numerical values in Table 3 indicate the blending amounts of each component (unit: parts by mass). Furthermore, a three-dimensional photofabricated product was prepared from the obtained curable composition for photofabrication. For the curable composition for photofabrication and the three-dimensional photofabricated product, the same evaluation as in Example 1 was performed. The results are presented in Table 4 below. FIG. 2 presents an optical microscope image (magnification: 50X) photographed during the observation in the cracking evaluation of Comparative Example 1.

### <Comparative Examples 3 and 4>

A curable composition for photofabrication was prepared in the same manner as in Example 1, except that the composition of the curable composition for photofabrication was changed as presented in Table 3 below. For the obtained curable composition for photofabrication, the same evaluation as in Example 1 was performed. The results are presented in Table 4 below. Note that it was impossible to shape the curable compositions for photofabrication in Comparative Examples 3 and 4 using a 3D printer due to a high viscosity of higher than 100,000 mPa·s.

### <Comparative Example 5>

A curable composition for photofabrication was prepared in accordance to the method described in Example 1 of Patent Document 3. Specifically, UDMA (70 parts by mass), ACMO (30 parts by mass), an inorganic filler composition C (100 parts by mass), TPO (3.0 parts by mass), and BHT (0.05 part by mass) were stirred and mixed to prepare a curable composition for photofabrication. Furthermore, a three-dimensional photofabricated product was prepared from the obtained curable composition for photofabrication. For the curable composition for photofabrication and the three-dimensional photofabricated product, the same evaluation as in Example 1 was performed. The results are presented in Table 4 below. FIG. 3 presents an optical microscope image (magnification: 50X) photographed during the observation in the cracking evaluation of Comparative Example 5.

**[Table 1]**

| | | (A) Radically-polymerizable monomer | | | (B) Inorganic filler | (C) Photopolymerization initiator | (D) Activating light absorbent | (E) Polymerization inhibitor | (F) Hyperbranched polymer | (G) Chain transfer agent |
|---|---|---|---|---|---|---|---|---|---|---|
| | | Polyfunctional monomer (a1) | Polyfunctional monomer (a2) | Other monomers | | | | | | |
| | 1 | UDMA (80) | - | 9G (20) | Composition A (150) | BTPO (1.4) | SS3 (0.7) | HQME (0.1) | HBPA (5) | - |
| | | | | | | | | BHT (0.1) | | |
| | 2 | UDMA (80) | - | 1G (20) | Composition A (150) | BTPO (1.4) | SS3 (0.7) | HQME (0.1) | HBPA (5) | - |
| | | | | | | | | BHT (0.1) | | |
| | 3 | UDMA (80) | 3G (20) | - | Composition A (150) | BTPO (1.4) | SS3 (0.7) | HQME (0.1) | HBPA (10) | - |
| | | | | | | | | BHT (0.1) | | |
| | 4 | UDMA (80) | 3G (20) | - | Composition A (150) | BTPO (1.4) | SS3 (0.7) | HQME (0.1) | HBPA (7) | - |
| | | | | | | | | BHT (0.1) | | |
| | 5 | UDMA (80) | 3G (20) | - | Composition A (150) | BTPO (1.4) | SS3 (0.7) | HQME (0.1) | HBPA (5) | - |
| | | | | | | | | BHT (0.1) | | |
| | 6 | UDMA (80) | 3G (20) | - | Composition A (150) | BTPO (1.4) | SS3 (0.7) | HQME (0.1) | HBPA (3) | - |
| | | | | | | | | BHT (0.1) | | |
| Example | 7 | UDMA (80) | 3G (20) | - | Composition A (150) | BTPO (1.4) | SS3 (0.7) | HQME (0.1) | HBPA (1) | - |
| | | | | | | | | BHT (0.1) | | |
| | 8 | UDMA (80) | 3G (20) | - | Composition A (150) | BTPO (1.4) | SS3 (0.7) | HQME (0.1) | HBPB (5) | - |
| | | | | | | | | BHT (0.1) | | |
| | 9 | UDMA (50) A-9300-1CL (30) | 3G (20) | - | Composition A (150) | BTPO (1.4) | SS3 (0.7) | HQME (0.1) | HBPA (5) | - |
| | | | | | | | | BHT (0.1) | | |
| | 10 | A-9300-1CL (50) | D-2.6E (30) 3G (20) | - | Composition A (150) | BTPO (1.4) | SS3 (0.7) | HQME (0.1) | HBPA (5) | - |
| | | | | | | | | BHT (0.1) | | |
| | 11 | UDMA (80) | 3G (20) | - | Composition A (50) | BTPO (1.4) | SS3 (0.7) | HQME (0.1) | HBPA (5) | - |
| | | | | | | | | BHT (0.1) | | |
| | 12 | UDMA (80) | 3G (20) | - | Composition A (110) | BTPO (1.4) | SS3 (0.7) | HQME (0.1) | HBPA (5) | - |
| | | | | | | | | BHT (0.1) | | |
| | 13 | UDMA (80) | 3G (20) | - | Composition A (233) | BTPO (1.4) | SS3 (0.7) | HQME (0.1) | HBPA (5) | - |
| | | | | | | | | BHT (0.1) | | |
| | 14 | UDMA (80) | 3G (20) | - | Composition A (150) | BTPO (3.0) | SS3 (0.7) | HQME (0.1) | HBPA (5) | - |
| | | | | | | | | BHT (0.1) | | |
| | 15 | UDMA (80) | 3G (20) | - | Composition A (150) | BTPO (0.3) | SS3 (0.7) | HQME (0.1) | HBPA (5) | - |
| | | | | | | | | BHT (0.1) | | |
| | 16 | UDMA (80) | 3G (20) | - | Composition A (150) | BTPO (1.4) | SS3 (2.0) | HQME (0.1) | HBPA (5) | - |
| | | | | | | | | BHT (0.1) | | |
| | 17 | UDMA (80) | 3G (20) | - | Composition A (150) | BTPO (1.4) | SS3 (0.02) | HQME (0.1) | HBPA (5) | - |
| | | | | | | | | BHT (0.1) | | |
| Example | 18 | UDMA (80) | 3G (20) | - | Composition A (150) | BTPO (1.4) | SS3 (0.7) | HQME (2.0) | HBPA (5) | - |
| | | | | | | | | BHT (2.0) | | |
| | 19 | UDMA (80) | 3G (20) | - | Composition A (150) | BTPO (1.4) | SS3 (0.7) | HQME (0.03) | HBPA (5) | - |
| | | | | | | | | BHT (0.03) | | |
| | 20 | UDMA (80) | 3G (20) | - | Composition A (150) | BTPO (1.4) | SS3 (0.7) | HQME (0.1) | HBPA (5) | - |
| | | | | | | | | BHT (0.1) | | |
| | 21 | UDMA (80) | 3G (20) | - | Composition A (150) | BTPO (1.4) | SS3 (0.7) | HQME (0.1) | HBPA (5) | MSD (0.3) |
| | | | | | | | | BHT (0.1) | | |
| | 22 | UDMA (80) | 3G (20) | - | Composition A (150) | BTPO (1.4) | SS3 (0.7) | HQME (0.1) | HBPA (5) | MSD (0.1) |
| | | | | | | | | BHT (0.1) | | |
| | 23 | UDMA (80) | 3G (20) | - | Composition A (150) | BTPO (1.4) | SS3 (0.7) | HQME (0.1) | HBPA (5) | MSD (0.01) |
| | | | | | | | | BHT (0.1) | | |

**[Table 3]**

| | | (A) Radically-polymerizable monomer | | | (B) Inorganic filler | (C) Photopolymerization initiator | (D) Activating light absorbent | (E) Polymerization inhibitor | (F) Hyperbranched polymer | Other components |
|---|---|---|---|---|---|---|---|---|---|---|
| | | Polyfunctional monomer (a1) | Polyfunctional monomer (a2) | Other monomers | | | | | | |
| Comparative Example | 1 | UDMA (80) | 3G (20) | - | Composition A (150) | BTPO (1.4) | SS3 (0.7) | HQME (0.1) | - | - |
| | | | | | | | | BHT (0.1) | | |
| | 2 | UDMA (80) | 3G (20) | - | Composition A (150) | BTPO (1.4) | SS3 (0.7) | HQME (0.1) | - | PMMA (5) |
| | | | | | | | | BHT (0.1) | | |
| | 3 | UDMA (80) | 3G (20) | - | Composition A (300) | BTPO (1.4) | SS3 (0.7) | HQME (0.1) | HBPA (5) | - |
| | | | | | | | | BHT (0.1) | | |
| | 4 | UDMA (10) | D-2.6E (70) 3G (20) | - | Composition B (275) | CQ (0.2) | - | - | HBPA (12) | DMBE (0.35) |
| | 5 | UDMA (70) | - | ACMO (30) | Composition C (100) | TPO (3.0) | - | BHT (0.05) | - | - |

**[Table 4]**

| | | Bending strength [MPa] | Cracking evaluation | Viscosity [mPa·s] | Presence/ absence of sedimentation | Solubility of (F) or PMMA [g] |
|---|---|---|---|---|---|---|
| Example | 1 | 121 | A3 | 7,000 | Absent | ≥ 10 |
| | 2 | 118 | A3 | 7,000 | Absent | ≥ 10 |
| | 3 | 150 | A1 | 30,000 | Absent | ≥ 10 |
| | 4 | 153 | A1 | 20,000 | Absent | ≥ 10 |
| | 5 | 159 | A1 | 12,000 | Absent | ≥ 10 |
| | 6 | 157 | A2 | 10,000 | Absent | ≥ 10 |
| | 7 | 152 | A3 | 9,000 | Absent | ≥ 10 |
| | 8 | 153 | A1 | 24,000 | Absent | ≥ 10 |
| | 9 | 159 | A1 | 21,000 | Absent | ≥ 10 |
| | 10 | 158 | A1 | 22,000 | Absent | ≥ 10 |
| | 11 | 142 | A1 | 3,000 | Absent | ≥ 10 |
| | 12 | 146 | A1 | 6,000 | Absent | ≥ 10 |
| | 13 | 160 | A2 | 45,000 | Absent | ≥ 10 |
| | 14 | 158 | A1 | 12,000 | Absent | ≥ 10 |
| | 15 | 149 | A1 | 11,000 | Absent | ≥ 10 |
| | 16 | 150 | A3 | 12,000 | Absent | ≥ 10 |
| | 17 | 160 | A1 | 12,000 | Absent | ≥ 10 |
| | 18 | 149 | A1 | 11,000 | Absent | ≥ 10 |
| | 19 | 159 | A2 | 12,000 | Absent | ≥ 10 |
| | 20 | 149 | A3 | 11,000 | Absent | ≥ 10 |
| | 21 | 162 | A1 | 12,000 | Absent | ≥ 10 |
| | 22 | 161 | A1 | 12,000 | Absent | ≥ 10 |
| | 23 | 159 | A1 | 12,000 | Absent | ≥ 10 |
| Comparative Example | 1 | 151 | C | 8,000 | Present | - |
| | 2 | 125 | C | 42,000 | Present | ≥ 10 |
| | 3 | - | - (Non-shapable) | ≥ 100,000 | Absent | ≥ 10 |
| | 4 | - | - (Non-shapable) | ≥ 100,000 | Absent | ≥ 10 |
| | 5 | 140 | C | 5,000 | Absent | - |

As presented in Table 4, the curable compositions for photofabrication in Examples 1 to 23 had a moderate viscosity and exhibited no inorganic filler sedimentation. Furthermore, the three-dimensional photofabricated products in Examples 1 to 23 exhibited high bending strength, and cracks observed on their surfaces were few in number and small in width.

In contrast, the curable composition for photofabrication in Comparative Example 1, having the same composition as in Examples 3 to 8 except for not containing the hyperbranched polymer, was prone to inorganic filler sedimentation. The three-dimensional photofabricated product in Comparative Example 1 had a large number of cracks on its surface. The curable composition for photofabrication in Comparative Example 2, having the same composition as in Examples 5 and 8 except that it contained polymethyl methacrylate instead of the hyperbranched polymer, had a higher viscosity and was more prone to inorganic filler sedimentation, compared to the curable composition for photofabrication in Examples 5 and 8. Furthermore, the three-dimensional photofabricated product in Comparative Example 2 had a low bending strength and showed a large number of cracks on its surface. The curable compositions for photofabrication in Comparative Examples 3 and 4, having an inorganic filler content of more than 250 parts by mass with respect to 100 parts by mass of the radically-polymerizable monomer, had a high viscosity of higher than 100,000 mPa·s and could not be shaped using a 3D printer. The curable composition for photofabrication in Comparative Example 5 was prepared according to the method described in Patent Document 3, and developed a large number of cracks on a three-dimensional photofabricated product obtained therefrom.

## Claims

1. A curable composition for three-dimensional photofabrication comprising:
100 parts by mass of a radically-polymerizable monomer (A) that is liquid at 25°C;
50 to 250 parts by mass of an inorganic filler (B);
0.05 to 5.0 parts by mass of a photopolymerization initiator (C) that absorbs activating light to generate radicals;
0.01 to 2.5 parts by mass of an activating light absorbent (D) that absorbs the activating light;
0.01 to 5.0 parts by mass of a polymerization inhibitor (E); and
0.3 to 10 parts by mass of a hyperbranched polymer (F) that is soluble in the radically-polymerizable monomer (A).

2. The curable composition for three-dimensional photofabrication according to claim 1, wherein the hyperbranched polymer (F) comprises:
a first unit structure having four bonding sites and represented by the following formula (I): 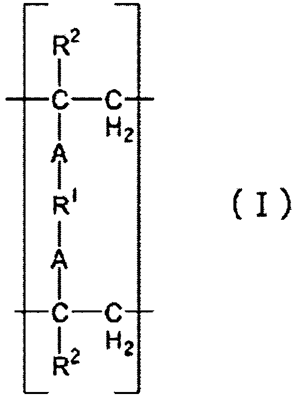
wherein A each independently represent a single bond for bonding C and R¹, >C=O, -O-, -COO-, or -COO-CH₂-, R¹ represents a divalent saturated aliphatic hydrocarbon group or a divalent aromatic hydrocarbon group, and R² each independently represent hydrogen atom or a methyl group; and
a second unit structure that is at least one of two unit structures represented by the following formulas (IIA) and (IIB): 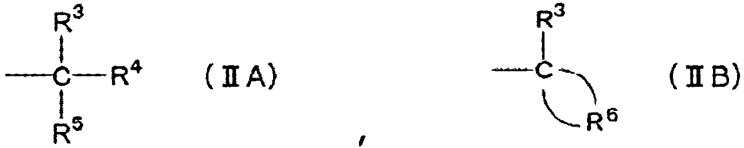
wherein R³, R⁴, and R⁵ each independently represent hydrogen atom, an alkyl group having 1 to 5 carbon atoms for forming a main chain, an alkoxycarbonyl group having 2 to 6 carbon atoms for forming a main chain, an aryl group, or a cyano group, and R⁶ represents an alkylene group having 4 to 10 carbon atoms for forming a main chain.

3. The curable composition for three-dimensional photofabrication according to claim 1, wherein a content of the hyperbranched polymer (F) is 2 to 10 parts by mass with respect to 100 parts by mass of the radically-polymerizable monomer (A).

4. The curable composition for three-dimensional photofabrication according to claim 1, wherein
90 mass% or more of the radically-polymerizable monomer (A) is composed of a polyfunctional (meth)acrylate type radically-polymerizable monomer (a) having two or more (meth)acryloyl groups in its molecule and having 5 to 25 atoms constituting a main chain of a divalent group interposed between the two or more (meth)acryloyl groups present in its molecule,
the polyfunctional (meth)acrylate type radically-polymerizable monomer (a) is composed of a polyfunctional (meth)acrylate type radically-polymerizable monomer (a1) having at least one selected from a group consisting of a urethane bond, a urea bond, and an isocyanurate ring; and another polyfunctional (meth)acrylate type radically-polymerizable monomer (a2), and
40 to 90 mass% of the radically-polymerizable monomer (A) is the polyfunctional (meth)acrylate type radically-polymerizable monomer (a1), and 10 to 60 mass% of the radically-polymerizable monomer (A) is the polyfunctional (meth)acrylate type radically-polymerizable monomer (a2).

5. The curable composition for three-dimensional photofabrication according to claim 1, further comprising 0.005 to 1.0 part by mass of a chain transfer agent (G).

6. The curable composition for three-dimensional photofabrication according to claim 1, which is for use as a liquid photocurable resin composition in a three-dimensional photofabrication in which, from three-dimensional shape data indicating a shape of a three-dimensional object, a height direction of the three-dimensional object is digitized and serialized, and two-dimensional shape data indicating sectional shapes of the three-dimensional object at serialized heights is generated, and then, using a vat photopolymerization method including irradiating a predetermined position of the liquid photocurable resin composition in a vat with activating light that is ultraviolet ray or visual light to selectively cure the liquid photocurable resin composition at the position, fabricated layers having shapes corresponding to the two-dimensional shapes at the heights are sequentially formed and stacked in accordance with the order of the serialization on the basis of the two-dimensional shape data, thereby obtaining a stacked body having a shape corresponding to the shape of the three-dimensional object.

7. A method for producing the curable composition for three-dimensional photofabrication according to claim 1, the method comprising:
mixing the radically-polymerizable monomer (A), the photopolymerization initiator (C), the activating light absorbent (D), the polymerization inhibitor (E), and the hyperbranched polymer (F) to prepare a liquid composition containing at least a part of the hyperbranched polymer dissolved therein, and
mixing the liquid composition and the inorganic filler (B) .

8. The method for producing the curable composition for three-dimensional photofabrication according to claim 7, wherein, when preparing the liquid composition, a hyperbranched polymer powder granule having a hydrodynamic average diameter of 2 to 40 nm as measured in tetrahydrofuran by a dynamic light scattering method is mixed.

9. A method for producing a three-dimensional photofabricated product by using the curable composition for three-dimensional photofabrication according to claim 1 as a liquid photocurable resin composition, the method comprising
a forming step in which, from three-dimensional shape data indicating a shape of a three-dimensional object, a height direction of the three-dimensional object is digitized and serialized, and two-dimensional shape data indicating sectional shapes of the three-dimensional object at serialized heights is generated, and then, using a vat photopolymerization method including irradiating a predetermined position of the liquid photocurable resin composition in a vat with activating light that is ultraviolet ray or visual light to selectively cure the liquid photocurable resin composition at the position, fabricated layers having shapes corresponding to the two-dimensional shapes at the heights are sequentially formed and stacked in accordance with the order of the serialization on the basis of the two-dimensional shape data, thereby obtaining a stacked body having a shape corresponding to the shape of the three-dimensional object.
